# EUROPEAN PATENT SPECIFICATION

(11) **EP 2 197 816 B1**
(45) Date of publication and mention of the grant of the patent: **24.10.2018**
(21) Application number: 08838757.6
(22) Date of filing: 22.09.2008
(51) Int. Cl.: C10G 2/00, C07C 1/04, B01J 29/072, B01J 29/24, B01J 29/04, B01J 29/46, B01J 29/68, B01J 29/14, C01B 3/38

(54) **METHOD OF DIRECT SYNTHESIS OF LIGHT HYDROCARBONS FROM NATURAL GAS**
VERFAHREN ZUR DIREKTEN SYNTHESE LEICHTER KOHLENWASSERSTOFFE AUS ERDGAS
PROCÉDÉ DE SYNTHÈSE DIRECTE D'HYDROCARBURES LÉGERS À PARTIR DE GAZ NATUREL

(30) Priority: 15.10.2007 KR 20070103677
(43) Date of publication of application: 23.06.2010
(73) Proprietor: Korea Research Institute of Chemical Technology, Yuseong-gu, Daejeon 34114 (KR)
(72) Inventor: BAE, Jong-Wook, Daejeon 305-343 (KR); KANG, Suk-Hwan, Daejeon 305-751 (KR); LEE, Yun-Jo, Daejeon 305-761 (KR); JUN, Ki-won, Daejeon 305-761 (KR)
(74) Representative: WSL Patentanwälte Partnerschaft mbB
(86) International application number: PCT/KR2008/005608
(87) International publication number: WO 2009/051353

(56) References cited:
- WO-A1-01/60773
- WO-A1-01/60773
- WO-A1-92/05869
- WO-A2-98/57743
- KR-A- 20040 051 953
- KR-B1- 100 482 646
- US-A- 4 340 503
- US-A- 4 622 308
- US-B1- 6 225 359
- Chong Wang ET AL: "Review of Directly Producing Light Olefins via CO Hydrogenation", Journal of Natural Gas Chemistry, 1 January 2003 (2003-01-01), pages 10-16, XP055113521, Retrieved from the Internet: URL:http://www.bjb.dicp.ac.cn/jngc/2003/03 -01/020902.pdf [retrieved on 2014-04-10]
- MULPURI JANARDANARAO: "Direct Catalytic Conversion of Synthesis Gas to Lower Olefins", INDUSTRIAL & ENGINEERING CHEMISTRY RESEARCH, AMERICAN CHEMICAL SOCIETY, US, vol. 29, no. 9, 1 September 1990 (1990-09-01), pages 1735-1753, XP002628374, ISSN: 0888-5885, DOI: 10.1021/IE00105A001
- C N Satterfield ET AL: "Fischer-Tropsch Synthesis in a Slurry Reactor: Preclpitated Iron-Copper-Potassium Catalyst", Ind. fng. Chem. Process Des. Dev., 1 January 1984 (1984-01-01), pages 849-851, XP055113569, Retrieved from the Internet: URL:http://pubs.acs.org/doi/pdf/10.1021/i2 00027a038 [retrieved on 2014-04-10]
- SUK-HWAN KANG ET AL: "Fischerâ Tropsch Synthesis Using Zeolite-supported Iron Catalysts for the Production of Light Hydrocarbons", CATALYSIS LETTERS, KLUWER ACADEMIC PUBLISHERS-PLENUM PUBLISHERS, NE, vol. 125, no. 3-4, 25 July 2008 (2008-07-25), pages 264-270, XP019640287, ISSN: 1572-879X, DOI: 10.1007/S10562-008-9586-2
- WANG Y N ET AL: "Kinetics modelling of Fischer-Tropsch synthesis over an industrial Fe-Cu-K catalyst", FUEL, IPC SCIENCE AND TECHNOLOGY PRESS, GUILDFORD, GB, vol. 82, no. 2, 1 January 2003 (2003-01-01), pages 195-213, XP027114676, ISSN: 0016-2361 [retrieved on 2003-01-01]
- HIRSA M. TORRES GALVIS ET AL: "Catalysts for Production of Lower Olefins from Synthesis Gas: A Review", ACS CATALYSIS, vol. 3, no. 9, 6 September 2013 (2013-09-06), pages 2130-2149, XP055113505, ISSN: 2155-5435, DOI: 10.1021/cs4003436
- YI-NING WANG ET AL.: 'Kinetics modelling of Fischer-Tropsch synthesis over an industrial Fe- Cu-K catalyst' FUEL vol. 82, 2003, pages 195 - 213, XP004385994

## Description

### [Technical Field]

The present invention relates to a method of direct synthesis of light hydrocarbons from natural gas which is capable of improving yield of light hydrocarbons production and carbon utilization efficiency through a series of process of preparing synthesis gas with a predetermined molar ratio of carbon monoxide to hydrogen by a combined reforming such as steam reforming of natural gas and carbon dioxide reforming of methane, performing Fischer-Tropsch reaction of the prepared synthesis gas in the presence of a specific catalyst, and recycling the main byproducts of methane and carbon dioxide as the feedstock of the combined reforming reaction.

### [Background Art]

The method of producing light hydrocarbons from a synthesis gas is similar to the production of light olefins from synthesis gas (STO; syngas to olefins). Fischer-Tropsch (F-T) synthesis in gas to liquid (GTL) process, which is a core process in STO reaction as well, was first developed in 1923 by German chemists of Fischer and Tropsch as a process of producing synthetic fuel from synthesis gas through gasification of coal.

The GTL process comprises three main units such as reforming of natural gas, F-T synthesis of synthesis gas and reforming of the product. In the F-T reaction, iron- and cobalt-based catalysts are generally used, and the reaction is performed under the temperature range of 200-350 °C and pressure range of 10-30 atm. The four major reactions of the F-T synthesis are as follows:
(a) Chain growth in F-T synthesis

| | |
|---|---|
| CO + 2H₂ → -CH₂- + H₂O | ΔH (227 °C) = -165 kJ/mol |

(b) Methanation

| | |
|---|---|
| CO + 3H₂ → CH₄ + H₂O | ΔH (227 °C) = -215 kJ/mol |

(c) Water gas shift reaction

| | |
|---|---|
| CO + H₂O → CO₂ + H₂ | ΔH (227 °C) = -40 kJ/mol |

(d) Boudouard reaction

| | |
|---|---|
| 2CO → C + CO₂ | ΔH (227 °C) = -134 kJ/mol |

For the F-T reaction, iron- and cobalt-based catalysts are mainly used. At the beginning of F-T process development, iron-based catalysts were mainly used. But, recently, cobalt-based catalysts that can be used at a much lower temperature are predominantly used in order to enhance the production of liquid fuel or wax and improve the conversion of synthesis gas. Iron-based catalysts have some advantages in that they are the least expensive of the F-T catalysts, produce a lesser amount of methane at high temperature, provide high selectivity for olefins, enable the production of light olefins or α-olefins that can be utilized as a feedstock in the chemical industry in addition to fuel, and produce many byproducts such as alcohols, aldehydes, ketones, etc., in addition to hydrocarbons.

For example, Sasol uses an iron-based catalyst comprising Cu and K as promoting components and prepared by precipitation method using SiO2 as a binder for low-temperature F-T reaction for the production of wax. For high-temperature F-T, Sasol uses a catalyst prepared by melting magnetite, K, alumina, MgO, etc., for the production of light olefins and gasoline. However, because the conventional F-T synthesis has some disadvantages that a wide-range of hydrocarbon distribution is obtained and selectivity for light hydrocarbons and olefins is low (selectivity for C₂-C₄ hydrocarbons = 30%; selectivity for olefins = 80%), the catalyst for STO reaction is required to have further catalytic properties for inhibiting carbon chain growth during the F-T synthesis or improving selectivity for light hydrocarbons through cracking of high boiling point compounds. To provide these properties, an F-T catalyst is designed to be combined with an additional component like a molecular sieve-structured material, a hybrid catalyst (hybrid of a methanol synthesis catalyst and a methanol-to-light olefin conversion catalyst) is adopted or the development of process engineering is applied to overcome the limitation of the F-T synthesis showing a wide hydrocarbon distribution intrinsically.

Along with the development of F-T catalyst, the reactor for the F-T process has been mainly developed by Sasol. The reactor has been developed from the fixed bed reactor to the circulating fluid bed reactor, to the fixed fluid bed, and to the slurry reactor. In 1985, the South African company, Sasol, developed the Synthol process and built an olefin and gasoline production plant (4.80 million tons per year). In 1994, the company introduced an advanced process and operated a plant with an annual production capacity of 450,000 tons. But, this process still has some disadvantages of the F-T synthesis, i.e. broad product distribution and low selectivity for olefins. As shown in Table 1, selectivity for C₂-C₄ hydrocarbons is about 30% and, selectivity for olefins of the hydrocarbons is about 80%. In addition, according to the Anderson-Schulz-Flory polymerization model, it is known that the maximum yield of C₂-C₄ hydrocarbons in the F-T synthesis reaction cannot exceed 56% due to the CH₂ chain growth mechanism.

**[Table 1]**

| Product selectivity (based on carbon) | LTFT | HTFT |
|---|---|---|
| CH₄ | 4 | 7 |
| C₂-C₄ olefins | 4 | 24 |
| C₂-C₄ paraffins | 4 | 6 |
| Gasoline | 18 | 36 |
| Middle distillate | 19 | 12 |
| Heavy oil and wax | 48 | 9 |
| Oxygenates | 3 | 6 |
| LTFT: Low-temperature F-T synthesis | | |
| HTFT: High-temperature F-T synthesis | | |

Recently, Dalian Institute of Chemical Physics (DICP, China) developed the K-Fe-MnO/Si-2 catalyst as an STO catalyst and reported that stable operation for 1,000 hours was possible without deactivation, with a CO conversion of 70% or more and a C₂-C₄ olefin selectivity of 71-74 % [Fuel Processing Technology 62 (2000) 161-172]. Efforts to improve yield and selectivity to light olefins from synthesis gas are made with respect to the development of catalyst. In this respect, many researches on remarkably improving selectivity of C₂-C₄ hydrocarbons by overcoming the limitation of the F-T reaction mechanism using a combination of zeolite and an F-T catalyst are reported recently [Catalysis Today 106 (2005) 143-148].

Since 1990s, European and U.S. companies as well as Sasol have also developed F-T technologies to develop a clean energy source and to cope with the high oil price era. Especially, the foreign companies possessing these technologies are limiting technical transfer of the GTL technology through a cartel. Commercialized F-T processes and main products of the processes are summarized in Table 2.

**[Table 2]**

| Reactor type | Process name | Company | Location | Startup | Plant capacity (t/a) | Main products |
|---|---|---|---|---|---|---|
| Fixed bed | ARGE | Sasol | Sasolburg | 1956 | Ca. 100x10³ | Wax, diesel, gasoline |
| | SMDS | Shell | Malaysia | 1994 | Ca. 500x10³ | Diesel, gasoline (wax, lubricant) |
| Circulating fluid bed | Synthol | Sasol | Secunda | 1985 | Ca. 480x10³ | Gasoline, light olefin (oxide) |
| Fixed fluid bed | SFFB | Sasol | Sasolburg | 1994 | Ca. 45x10³ | Gasoline, light olefin (oxide) |
| Slurry | SSBR | Sasol | Sasolburg | 1994 | Ca. 100x10³ | Wax, diesel, gasoline |
| ARGE: Arbeitsgemeinschaft Lurgi-Ruhrchemie | | | | | | |
| SMDS: Shell Middle Distillate Synthesis | | | | | | |
| SFFB: Sasol Fixed Fluidized Bed | | | | | | |
| SSBR: Sasol Slurry Bed Reactor | | | | | | |

The existing technologies to produce olefins from synthesis gas are based on the F-T synthesis, and olefins are prepared generally by the post-treatment process through dehydrogenation of hydrocarbons generated from F-T synthesis or direct dehydrogenation during the F-T reaction using an improved bifunctional catalyst in order to obtain olefins directly from synthesis gas. Although different catalysts and separation processes are used depending on the targeted products, a process of producing light olefins simultaneously along with fuel, e.g., LPG, constitutes the majority. For example, ExxonMobil's AGC-21, Oryx process based on the Sasol process, Synthroleum's S-2 process characterized by reformer technology, and the like are commercialized for the production of olefins from synthesis gas. Further, the DOE is known to be carrying out an IGCC-related process research through the Vision 21 program, aiming at a production cost of 15-20 $/bbl with respect to the coal to liquid (CTL) process. Of the zeolite-based catalyst techniques related to the production of olefins from synthesis gas, U.S. Patent No. 4,283,306 (1981, DuPont) is the most cited. It encompasses not only the production of olefins, but also the selective production of aromatic compounds utilizing the structural characteristics of the zeolite molecular sieve catalyst. Of the Fe-Co-based catalyst techniques related to the production of olefins from synthesis gas, U.S. Patent No. 4,151,190 (1979, Dow Chemical) is the most cited. It discloses the production of ethane, ethylene, dimethyl ether, and the like using an olefin conversion catalyst such as Pt. Of the processes for producing olefins from synthesis gas, U.S. Patent No. 4,423,265 (1983, Mobil Oil) is the most cited. It discloses the production of highly pure gasoline products using ZSM-5 zeolite as catalyst, which is effectively utilized for the synthesis of liquid hydrocarbons through the F-T process.

For the production of synthesis gas from natural gas, the following generally known processes are used. Researches are actively carried out on the process in order to obtain an adequate molar ratio of H₂/CO for the F-T synthesis.
(e) Steam reforming of methane (SRM)

| | |
|---|---|
| CH₄ + H₂O → 3H₂ + CO | ΔH = 226 kJ/mol |

(f) Carbon dioxide reforming of methane (CDR)

| | |
|---|---|
| CH₄ + CO₂ → 2H₂ + 2CO | ΔH = 261 kJ/mol |

(g) Partial oxidation of methane (POx)

| | |
|---|---|
| CH₄ + 0.5O₂ → 2H₂ + CO | ΔH = -44 kJ/mol |

KR 20040051953 A discloses a catalyst for reforming natural gas prepared by coprecipitation method which is a novel nickel-based reforming catalyst widely applied to the reforming of the natural gas such as CO₂, O₂, H₂O and mixture gases thereof and prevents inactivation of the catalyst due to carbon precipitation.

WO 01/60773 A1 discloses a method of operating a pre-reformer in a gas-to-liquids plant for the efficient production of synthesis gas to be used in producing liquid hydrocarbons preferably through a Fischer-Tropsch synthesis.

US 4,340,503 A discloses a catalyst comprising a silicalite support having iron present in the range of from about 5 to about 25 percent by weight and having potassium present in an amount not less than about 0.2 percent by weight, wherein the catalyst is used to convert synthesis gas to C₂-C₄ olefins.

WO 92/05869 A1 discloses iron-zinc based catalysts containing copper and a Group I metal promoter, and their use in a process for the conversion of synthesis gas to alpha olefins, wherein the iron:zinc atomic ratio of the catalyst is at least about 5:1, and the alkali metal:copper atomic ratio is at least about 2:1.

WO 98/57743 A2 discloses a method forming modified molecular sieves comprising a catalytically effective amount of a Fischer-Tropsch catalyst selected from the group consisting of iron, cobalt, nickel, chromium, manganese, and rhodium.

US 4,622,308 A discloses a method fo the production of hydrocarbons from synthesis gas by using an iron-containing Fischer-Tropsch catalyst, a zeolite and at least one metal selected from the group consisting of ruthenium, rhodium, platinum, palladium, iridium, cobalt and molybdenum, wherein the zeolite may be selected from erionite, ofretite and ferrierite.

C. N. Satterfield, et al. disclosed in 1984 a Fischer-Tropsch synthesis in a slurry reactor using a precipitated iron-copper-potassium catalyst (cf. Ind. Eng. Chem. Process Des. Dev., pages 849 - 851).

Mulpuri Janardanarao disclosed in 1990 a ZSM-5 or mordenite based catalyst in "Direct Catalytic Conversion of Synthesis Gas to Lower Olefins" (cf. Industrial & Engineering Chemistry Research, American Chemical Society, vol. 29, no. 9, pages 1735 - 1753).

Chong Wang, et al., disclosed in 2003 a "Review of Directly Producing Light Olefins via CO Hydrogenation" in the Journal of Natural Gas Chemistry (cf. e.g. pages 10 - 16).

Suk-Hwan Kang, et al. disclosed in 2008 a Fischer-Tropsch synthesis using zeolite-supported iron catalysts for the production of light hydrocarbons (cf. Catalysis Letters, Kluwer Academic Publishers, vol. 125, no. 3-4, pages 264 - 270).

### [Disclosure]

### [Technical Problem]

There is provided a method for improving productivity and carbon utilization efficiency in the synthesis of light hydrocarbons from natural gas by omitting the methanol synthesis route and the hydrotreating process of F-T products in the related art by using a specific catalyst.

### [Technical Solution]

The present invention relates to a preparation method of light hydrocarbons (C₂-C₄) from natural gas comprising the sequential steps of:
producing gas having a molar ratio of carbon monoxide and hydrogen being maintained at 1 :1.5-2.5, by combined reforming in which steam reforming of natural gas and carbon dioxide reforming of methane are carried out simultaneously in the presence of Ni/alumina catalyst;
separating of CH₄/CO₂-rich, unreacted gas, and H₂/CO-rich-gas in the produced gas;
recycling the CH₄/CO₂-rich-unreacted gas to the combined reforming reaction;
producing C2-C4-hydrocarbons, methane and carbon dioxide as byproducts by Fischer-Tropsch reaction at 200-700°C of carbon monoxide and hydrogen in H₂/CO-rich gas in the presence of a Fe-Cu-K/zeolite catalyst, wherein said catalyst has a specific surface area of 150-300 m²/g, comprises 10-70 wt% of Fe, 1-10 wt% of Cu and 1-10 wt% of K, based on the zeolite contents, and has been reduced under hydrogen atmosphere; and
separation of the byproducts, methane and carbon dioxide, and recycling them to the combined reforming reaction unit,
wherein the zeolite is ferrierite.

### [Advantageous Effects]

With respect to the technical development for selective production of light olefins, which is a fundamental feedstock in the petrochemical industry, and light hydrocarbons such as LPG to cope with the recent drastic increase of oil price, simplification of processes and development of catalyst which is effective in the production of light hydrocarbons may be an important competitive factor. In
particular, because the improvement of catalytic activity for the production of light hydrocarbons and the reaction process may lead to the improvement of thermal efficiency and carbon utilization efficiency of the overall process. The design of an optimized process, preparation method of light hydrocarbons and the catalyst for the production of light hydrocarbons showing stable selectivity and low deactivation rate provided herein will greatly contribute to the development of a cost-effective process for the synthesis of light hydrocarbons.

### [Description of Drawings]

The above and other features of the present invention will now be described in detail with reference to certain example embodiments thereof illustrated in the accompanying drawings which are given herein below by way of illustration only, and thus are not limited to the present invention, and wherein:
Fig. 1 schematically illustrates a reaction process for selectively producing light hydrocarbons from natural gas in accordance with the present invention; and
Fig. 2 illustrates conversion of carbon monoxide with time on stream during the Fischer-Tropsch reaction carried out in the presence of a Fe-Cu-K/ ferrierite catalyst in accordance with the present invention.

### [Best Mode]

In accordance with the present invention, through a combined reforming in which steam reforming of natural gas (CH₄) (SRM; steam reforming of methane) and carbon dioxide reforming of methane (CDR) are carried out simultaneously, the molar ratio of the synthesis gas product of carbon monoxide and hydrogen is maintained within a predetermined range. Then, the synthesis gas product is subjected to a Fischer-Tropsch (F-T) reaction in the presence of a Fe-Cu-K/ ferrierite catalyst according to the invention to prepare light hydrocarbons. Methane and carbon dioxide, which are produced as byproducts, are separated and recycled to the combined reforming process.

The molar ratio of carbon monoxide and hydrogen used in the F-T reaction is 1 : 1.5-2.5. If the amount of hydrogen is less, one-pass conversion of carbon monoxide may be reduced. And, if the amount of hydrogen is more, selectivity for methane may be increased. Hence, the molar ratio of carbon monoxide and hydrogen in the synthesis gas produced from the natural gas is maintained at 1 : 1.5-2.5. This range of molar ratio is more effective for the F-T synthesis than that of the synthesis gas produced from the steam reforming of natural gas only, which is 1 : 3.0-3.5. As a result, production rate of byproducts can be reduced and carbon monoxide conversion can be improved. This is attained by carrying out recycling the byproducts of carbon dioxide and methane and performing the combined reforming of steam reforming and carbon dioxide reforming simultaneously.

By performing F-T reaction in the presence of a specific catalyst, with the molar ratio of carbon monoxide and hydrogen being maintained within the aforesaid range, the production yield of light hydrocarbons is improved and the production of byproducts is significantly reduced as compared when using conventional synthesis gas. As a result, the cost of processing the byproducts can be reduced and the simplification of process can be attained. That is, by using a synthesis gas with an adequate molar ratio, productivity to the light hydrocarbons from natural gas can be improved.

In the F-T reaction for producing liquid hydrocarbons from synthesis gas, use of an iron-based catalyst generally gives a broad product distribution and a low selectivity for olefins. It has been reported that the selectivity for C₂-C₄ hydrocarbons is about 30% and the selectivity for olefins in C₂-C₄ hydrocarbons is about 80%. Further, according to a calculation using the Anderson-Schulz-Flory polymerization model, it is known that the maximum yield of C₂-C₄ hydrocarbons in the F-T synthesis cannot exceed 56% because of the CH₂ chain growth mechanism. Accordingly, with respect to the production of light hydrocarbons from synthesis gas, a thermal cracking of heavy hydrocarbons with high boiling point, a cracking of catalyst using a zeolite-based acid catalyst, and a dehydrogenation reaction are presented to improve selectivity for light olefins and light hydrocarbons.

In the F-T using the conventional synthesis gas, the production of heavy hydrocarbons tends to predominate, resulting in low yield of light hydrocarbons. Thus, an additional process of thermal cracking of the heavy hydrocarbons or cracking with the catalyst is inevitable, which requires further fuel consumption and increased utility cost. The present invention solves this problem by providing a new catalyst system for the production of light hydrocarbons and providing a method for improving production yield of light hydrocarbons from synthesis gas directly. That is, the present invention aims at solving the problem by preparing a synthesis gas from natural gas and performing F-T reaction using the prepared synthesis gas. Namely, the present invention is not simply a combination of previously known reactions under optimized reaction conditions, but a specific catalyst is used to optimize the production process.

As described earlier, each step of the process is widely known in the related art, but a catalyst, reaction conditions, separation method, recycling method of the product, and a combined process related thereto with a view to attain the effect provided by the present invention are rarely known in the related art. Therefore, the present invention aims to produce light hydrocarbons effectively by improving the existing process.

Further, the present invention provides a catalyst and a process different from existing ones for improved productivity of light hydrocarbons through superior one-pass conversion using a specific catalyst for the production of light hydrocarbons and for an effective recycling scheme of the byproducts such as methane and carbon dioxide. By selectively recycling the byproducts in the combined reforming to produce a synthesis gas suitable for the production of light hydrocarbons, carbon utilization efficiency is improved and the process required for the cracking of heavy hydrocarbons with high boiling point can be omitted, thereby resulting in improvement of energy efficiency of the overall process.

Hereinafter, the present invention will be described in more detail.

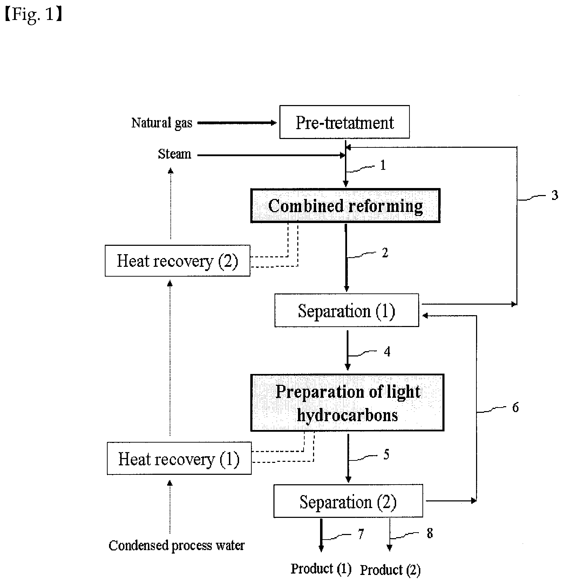

Fig. 1 schematically illustrates a reaction process for selectively producing light hydrocarbons from natural gas in accordance with the present invention. In the figure, "1" denotes the flow of natural gas, steam, and carbon dioxide and methane recycled after the production of light hydrocarbons to a combined reforming process; "2" denotes the flow of carbon monoxide and hydrogen produced from the combined reforming and other unreacted substance; and "3" denotes the flow of carbon dioxide and methane separated through a separation process (1) following the combined reforming. "4" denotes the flow of hydrogen and carbon monoxide separated from the separation process (1) for the production of light hydrocarbons; "5" denotes the flow of the produced light hydrocarbons, other unreacted substance and byproducts (methane, carbon dioxide and heavy hydrocarbons with high boiling point) introduced to a separation process (2); and "6" denotes the flow of the unreacted substance and the byproducts (methane and carbon dioxide) separated through the separation process (2) and introduced again to the separation process (1). And, "7" denotes the flow of C₂-C₄ light hydrocarbons, which are the main reaction products; and "8" denotes the flow of other high boiling point hydrocarbon products including naphtha, diesel, alcohol and wax.

The process of preparing light hydrocarbons from natural gas in accordance with the present invention will be described in detail with reference to the schematic diagram Fig. 1.

Combined reforming is carried out in which steam reforming of natural gas and carbon dioxide reforming of methane are performed simultaneously in the presence of a Ni/alumina catalyst, with the molar ratio of carbon monoxide and hydrogen being maintained at 1 : 1.5-2.5.

The combined reforming consists of steam reforming of natural gas (methane; CH₄) (SRM) and carbon dioxide reforming of methane (CDR). During the combined reforming, the molar ratio of carbon monoxide and hydrogen is be maintained at 1 : 1.5-2.5. If the amount of hydrogen is less than 1.5 mol, one-pass
conversion of carbon monoxide may decrease, thereby resulting in decreased production of light hydrocarbons. Meanwhile, if it exceeds 2.5 mol, selectivity for methane may increase excessively. Hence, the aforesaid range is preferred.

The combined reforming may be carried out by increasing the amount of steam or further supplying oxygen in order to inhibit the catalyst deactivation due to carbon deposition and to improve carbon monoxide production. The oxygen may be supplied in an amount of 0.02-0.30 mol, based on 1 mol of carbon dioxide. If the amount of oxygen addition is less than 0.02 mol, the effect of addition may be trivial and the carbon monoxide production may not increase sufficiently. Meanwhile, if the amount of oxygen addition exceeds 0.30 mol, conversion of carbon dioxide may decrease as carbon dioxide is generated again through oxidation of carbon monoxide. Hence, the aforesaid range is preferred. And, the water produced during the preparation of light hydrocarbons may be utilized effectively in the SRM reaction through a heat exchange process (1) and (2) in Fig. 1 in order to improve energy efficiency of the overall process.

The catalyst used in the combined reforming may be a Ni/alumina catalyst presented by the applicant of the present invention [Korean Patent No. 0482646]. Preferably, it may be prepared by coprecipitation method or supporting the active component of Ni on an alumina support which is pre-treated with Ce-ZrO₂, so that the active component can be uniformly distributed, or further treating with an alkali metal or alkaline earth metals, such that deactivation by carbon deposition can be inhibited and long-term stability can be achieved. Specifically, the Ni/alumina catalyst may have the following Chemical Formula 1.

[Chemical Formula 1] NiₓCe_{y}Zr_{z}Oₓ+_{2(y+z)}/Al₂O₃

wherein x + y + z = 1.0, x is 0.04-0.45, and y is 0.01-0.96.

The support may be γ- or θ-alumina.

The catalyst may be reduced at 500-1,000 °C before it is used for the combined reforming. The combined reforming may be performed at 600-1,000 °C, 0.5-30 atm, and a space velocity of 1,000-500,000 h⁻¹. Oxygen may be further used in the reforming reaction. In this case, it may be used in an amount of 0.02-0.30 mol based on 1 mol of carbon dioxide.

The combined reforming provides a CH₄ conversion of 80-95 % and a CO₂ conversion of 60-90%.

Next, Fischer-Tropsch reaction of carbon monoxide and hydrogen is carried out in the presence of a Fe-Cu-K/ ferrierite catalyst according to the invention in order to produce light hydrocarbons (C₂-C₄) and byproducts such as methane and carbon dioxide.

The catalyst comprises active metal components of Fe, Cu and K. The contents of the active metal components are: Fe = 10-70 wt%, Cu = 1-10 wt%, and K = 1-10 wt% based on zeolite. If the content of Fe is below 10 wt%, the number of active sites for the production of light hydrocarbons may decrease, thereby resulting in decreased one-pass conversion of carbon monoxide. And, if it exceeds 70 wt%, specific surface area of the catalyst may decrease because of the blocking of pores, distribution of Fe may be nonuniform, and, as a result, reaction activity for the production of light hydrocarbons may be insufficient. If the content of Cu, which is added to improve the reducing property of Fe, is below 1 wt%, the catalytic activity may not increase sufficiently. And, if it exceeds 10 wt%, production of byproducts such as alcohols may increase or the number of catalytic active sites may decrease because of decreased Fe content. If the content of K is less than 1 wt%, selectivity for methane may increase because the addition effect is insignificant. And, if it exceeds 10 wt%, one-pass conversion of carbon monoxide may decrease because of decreased Fe active sites.

0-20 wt% of Al may be further used as an additional component, based on the zeolite. Here, Al serves as a structural modifier which improves the distribution property of the Fe and Cu components, thereby increasing the number of active sites for the production of light hydrocarbons and improving specific surface area. If the content of Al exceeds 20 wt%, increasing the number of active sites for the production of light hydrocarbons may decrease because of decreased Fe content, and, as a result, one-pass conversion of carbon monoxide may decrease.

The ferrierite zeolite used as the support may be one commonly used in the related art, having a specific surface area of 200-500 m²/g and a Si/ Al molar ratio of 2-200. If the specific surface area is smaller than 200 m²/g, the number of catalytic active sites may decrease because of decreased dispersion of Fe, Cu and K components, and, as a result, one-pass conversion of carbon monoxide may decrease. And, if it exceeds 500 m²/g, specific surface area of the catalyst may decrease because the micropores may be blocked during the impregnation of the Fe, Cu and K components. Hence, the aforesaid range is preferred. Further, the zeolite may be pre-treated with proton, a single metal precursor selected from IA, IIA, Zr, P and La or a bimetal precursor by ion exchange or impregnation method, and then baked at 300-600 °C. If the molar ratio of the metals used for the pre-treatment exceeds 50 mol based on Al, the reactivity of catalytic cracking of heavy olefins with high boiling point may decline because of excessive removal of acid sites of the zeolite, thereby resulting in decreased selectivity for light hydrocarbons.

The Fe-Cu-K/ ferrierite catalyst according to the invention has a specific surface area of 150-300 m²/g. If the specific surface area of the catalyst is smaller than 150 m²/g, the number of active sites decreases, thereby resulting in reduced one-pass conversion of carbon monoxide. And, if it exceeds 300 m²/g, the catalyst may be quickly deactivated because of declined thermal stability of the catalyst, or catalytic activity may decrease or byproducts may be produced excessively because of decreased Fe-Cu-K content. Hence, the aforesaid range is preferred.

The catalyst may be prepared by the method commonly used in the related art. For example, it may be prepared by impregnation or coprecipitation. A detailed description will be given below.

For the iron precursor, a Fe(II) or Fe(III) precursor such as iron nitrate hydrate (Fe(NO₃)₃·9H₂O), iron acetate (Fe(CO₂CH₃)₂), iron oxalate hydrate(Fe(C₂O₄)₃·6H₂O), iron acetylacetonate (Fe(C₅H₇O₂)₃), iron chloride (FeCl₃), etc. may be used. The copper component added to improve the reducing property of iron may be selected from acetate, nitrate or chloride precursor. The potassium precursor used to improve olefin selectivity may be K₂CO3 or KOH. Further, Al may be added to improve the dispersion of Fe-Cu component. To this end, acetate or nitrate precursor may be used.

Pre-treated zeolite may be impregnated with the Fe, Cu, Al and K precursors simultaneously or consecutively, and calcined at 300-700 °C to obtain the catalyst. If the calcination temperature is below 300 °C, the dispersion of the active components may decline because the Fe, Cu, Al and K components may not be oxidized. And, if the calcination temperature exceeds 700 °C, the number of active sites may decrease due to aggregation of the Fe, Cu, Al and K components.

Alternatively, pre-treated zeolite may be suspended in a metal precursor solution of Fe, Cu and Al precursors, which is subjected to coprecipitation in an aqueous solution of pH 7-8, followed by aging at 40-90 °C. The resulting precipitate is filtered and washed to obtain the catalyst. During the coprecipitation, a basic precipitant may be used to maintain the pH at 7-8. Specifically, the basic precipitant may be sodium carbonate, calcium carbonate, ammonium carbonate or ammonia water. The catalyst may be aged for 0.1-10 hours, preferably for 0.5-8 hours. The presented aging time is of help to form a superior iron-based catalyst. If the aging time is shorter than 0.5 hour, dispersion of the Fe, Cu and Al components may decrease, unfavorably for the F-T reaction. And, an aging time exceeding 10 hours is economically unfavorable, because the number of active sites decreases due to increased Fe, Cu and Al particle size and the catalyst preparation time increases. The K component is supported on the hybrid catalyst of Fe-Cu-Al catalyst and ferrierite zeolite prepared by the coprecipitation at a content of 1-10 wt% to prepare the final catalyst for the production of light hydrocarbons from synthesis gas. Thus prepared catalyst is dried in an oven of 100 °C or higher for about a day, and may be calcined at 300-700 °C, preferably at 400-600 °C, to be used as a catalyst for the production of light hydrocarbons from synthesis gas.

The catalyst for the production of light hydrocarbons is used in the catalytic reaction in a fixed bed, fluidized bed or slurry reactor at 200-700 °C, after being reduced under hydrogen atmosphere. Reaction using the pre-reduced catalyst for the production of light hydrocarbons is carried out under a reaction condition similar to that of general STO synthesis reaction. Specifically, the reaction may be carried out at 250-500 °C, 5-60 kg/ cm² and a space velocity of 500-10,000 h⁻¹.

Next, the byproducts of methane and carbon dioxide are separated and recycled to the combined reforming reaction.

Yield of the light (C₂-C₄) hydrocarbons is maintained at 15-55 carbon mol%, yield of light olefins (C₂-C₄) at 10-45 carbon mol%, and CO conversion at 70-99 carbon mol%.

Fig. 1 illustrates the reaction process for producing light hydrocarbons from natural gas in accordance with the present invention.

First, the reactant natural gas is purified by pre-treatment (desulfurization and removal of heavy metal components) in order to prevent deactivation of catalyst in the following process. At the very beginning of reaction without recycle stream, the pre-treated natural gas stream 1 is subjected to SRM reaction in the presence of a nickel-based catalyst to produce synthesis gas. At this first stage, the theoretical H₂/CO ratio of the synthesis gas is 3 or larger, depending on whether a water gas shift reaction is accompanied [see the reaction formula (e)]. In general, an optimum H₂/CO ratio n the F-T synthesis is about 2. If the ratio is smaller, one-pass conversion of carbon monoxide may decrease. And, if the ratio is larger, selectivity for methane tends to increase. A gas stream 6 consisting of unreacted components and the byproducts of methane and carbon dioxide is separated by pressure swing adsorption (PSA) or membrane separation (separation process (1)) into a CH₄/CO₂-rich gas stream 3 and recycled to the combined reforming process, where the SRM reaction [reaction formula (e)] and the CDR reaction [reaction formula (f)] occur simultaneously. Because the H₂/CO ratio in the CDR reaction is about 1 [see the reaction formula (f)], synthesis gas suitable for the F-T reaction, in which the H₂/CO ratio is about 2, can be prepared through the combined reforming of SRM and CDR reactions. Here, in order to prevent catalyst deactivation caused by accumulation of carbon mainly, a small amount of oxygen may be added during the combined reforming reaction. A gas stream 2 produced from the combined reforming is separated in the separation process (1) into a CH₄/CO₂-rich unreacted gas stream 3 for recycling, and an H₂/CO-rich gas stream 4 is used for the production of light hydrocarbons. Accordingly, the combined reforming reaction may comprise an SRM (steam reforming of methane) process and a CDR (carbon dioxide reforming of methane) process, which are carried out simultaneously.

Thus produced products are transferred via a stream 5. The stream 5 includes methane, carbon dioxide, C₂-C₄ light hydrocarbons, C5⁺ liquid hydrocarbons and unreacted H₂/CO. These components may be separated by a general separation method (separation process (2)). The unreacted components and the CH₄/CO₂-rich component is introduced again into the separation process (1) by the stream 6, and the C₂-C₄ component is separated via a stream 7 (product 1). Of the C₂-C₄ light hydrocarbon component, olefins may be used for the synthesis of polymers (polyethylene, polypropylene, etc.) or other chemicals after further purification, and C₂-C₄ paraffins may be converted to C₂-C₄ olefin through further thermal cracking or may be used as heat source of the combined reforming process or as LPG. The C₅+ hydrocarbons (product 2) separated as a stream 8 may be further converted to C₂-C₄ olefins through catalytic thermal cracking or may be used as gasoline, naphtha, etc. Water produced by condensation during the production of light hydrocarbons may be sequentially heat exchanged through heat recovery (1) and (2) in the processes of light hydrocarbon production process and combined reforming process, and may be effectively used as a reactant of steam in the combined reforming reaction, thereby improving thermal efficiency of the overall process.

### [Mode for Invention]

### Example 1 (inventive): Preparation of synthesis gas from natural gas

Cerium acetate (Ce-acetate) and zirconium nitrate (Zr-nitrate) were supported on γ-alumina by impregnation, so that the Ce/Zr ratio was 0.25 based on weight. Calcination was carried out at 900 °C for 6 hours to prepare a Ce-ZrO₂/θ-Al₂O₃ support.

Nickel nitrate (Ni(NO₃)₂·6H₂O) was supported on the prepared Ce-ZrO₂/θ-Al₂O₃ support, with 12 wt% based on the support, and calcinations was carried out at 550 °C for 6 hours to prepare a Ni_{0.40}Ce_{0.12}Zr_{0.48}O₂/θ-Al₂O₃ catalyst. Prior to carrying out combined reforming, 1.0 g of the catalyst was packed in an Incolloy 800 H reactor, and reduced at 700 °C for 3 hours, under hydrogen (5 vol% H2/N2) atmosphere.

Reaction was carried out at 800 °C, 1 kg/cm², and a space velocity 12,420 L (CH₄)/kg cat/hr, while supplying reactants at a fixed molar ratio of CH₄ : (CO₂/H₂O/2O₂) : N₂ = 1 : 1.2 : 1. The reaction was carried out while varying the proportions CO₂/H₂O and O₂/H₂O. Average value was taken in the range where the catalytic activity was stabilized. The result is given in the following Table 3.

**[Table 3]**

| CO₂/H₂O (molar ratio) | O₂/H₂O (molar ratio) | CH₄ conversion | CO₂ conversion | H₂/CO (molar ratio) |
|---|---|---|---|---|
| 1.00 | 0.00 | 92.3 | 77.5 | 1.7 |
| 0.75 | 0.13 | 90.5 | 79.3 | 1.8 |
| 0.50 | 0.25 | 92.5 | 65.7 | 2.1 |

### Preparation of light hydrocarbons from synthesis gas

### Example 2 (inventive): Preparation of catalyst for production of light hydrocarbons (impregnation)

Ammonia type ferrierite zeolite (Si/Al = 10; specific surface area = 400 m²/g) was calcined at 600 °C to prepare proton type ferrierite zeolite. The resultant proton type ferrierite zeolite of 5 g was mixed with a solution in which iron nitrate hydrate (Fe(NO₃)₃·9H₂O), as iron precursor, copper nitrate (Cu(NO₃)₂·6H₂O), as copper precursor, and K₂CO₃, as potassium precursor, were dissolved in 60 mL of deionized water, and was agitated at room temperature for over 12 hours to prepare a impregnated catalyst. The impregnated catalyst was dried at 100 °C over 12 hours and calcined at 500 °C for 5 hours under air atmosphere. Thus prepared catalyst had a composition: 20 wt% Fe/2 wt% Cu/4 wt% K/ferrierite, based on metal weight. Specific surface area of the catalyst was 227 m²/g.

Prior to reaction, 0.3 g of the prepared catalyst was packed in a 1/2 inch stainless steel fixed bed reactor, and reduced at 450 °C for 12 hours under hydrogen (5 vol% H₂/He) atmosphere. Reaction was carried out at 300 °C, 10 kg/cm², and a space velocity of 2,000 L/kg cat/hr, while supplying reactants at a fixed molar ratio of carbon monoxide : hydrogen : argon (internal standard) = 63.2 : 31.3 : 5.5. Average value was taken after the catalytic activity was stabilized, that is, after 50 hours on stream. The result is given in Table 4.

### Example 3 (comparative)

A catalyst was prepared in the same manner as Example 2. Ammonia type ZSM-5 zeolite (Si/Al = 25, specific surface area = 350 m²/g) was calcined at 600 °C to prepare proton type ZSM-5 zeolite. The prepared catalyst had a composition: 20 wt% Fe/2 wt% Cu/4 wt% K/ZSM-5, based on metal weight. Specific surface area of the catalyst was 265 m²/g. Reduction and reaction were carried out in the same manner as Example 2. Average value was taken after the catalytic activity was stabilized, that is, after 50 hours on stream. The result is given in Table 4.

### Example 4 (comparative)

A catalyst was prepared in the same manner as Example 2. Sodium type Y-zeolite (Si/Al = 2.5, specific surface area = 600 m²/g) was ion exchanged with ammonia and calcined at 600 °C to prepare proton type Y-zeolite. The prepared catalyst had a composition: 20 wt% Fe/2 wt% Cu/4 wt% K/Y-zeolite, based on metal weight. Reduction and reaction were carried out in the same manner as Example 2. Average value was taken after the catalytic activity was stabilized, that is, after 50 hours on stream. The result is given in Table 4.

### Example 5 (comparative)

A catalyst was prepared in the same manner as Example 2. Mordenite zeolite (Si/Al = 6, specific surface area = 350 m²/g) was pre-treated and calcined at 600 °C to prepare proton type Mordenite zeolite. The prepared catalyst had a composition: 20 wt% Fe/2 wt% Cu/4 wt% K/Mordenite, based on metal weight. Reduction and reaction were carried out in the same manner as Example 2. Average value was taken after the catalytic activity was stabilized, that is, after 50 hours on stream. The result is given in Table 4.

### Example 6 (inventive): Preparation of catalyst for production of light hydrocarbons (coprecipitation)

Ammonia type ferrierite zeolite (Si/Al = 10; specific surface area = 400 m²/g) was calcined at 600 °C to prepare proton type ferrierite zeolite. The resultant proton type ferrierite zeolite of 1.2 g was agitated in a three-neck flask in a slurry phase using deionized water of 70 °C. A solution of metal mixture in which 21 g of iron nitrate hydrate (Fe(NO₃)₃·9H₂O), as iron precursor, 1.5 g of copper nitrate hydrate (Cu(NO₃)₂·6H₂O), as copper precursor, and 4.9 g of aluminum nitrate hydrate (Al(NO₃)₃·9H₂O), as alumina precursor, were dissolved in 400 mL of deionized water was prepared. A solution in which 19.5 g of potassium carbonate was dissolved in 400 mL of deionized water was used as a precipitant. To 200 mL of deionized water containing the ferrierite zeolite in a slurry phase in a 2,000 mL flask, the metal mixture solution and the precipitant solution prepared above were slowly added at 70 °C separately, and the final pH was maintained at 7.5-8.0. The mixture solution was agitated at 70 °C for about 3 hours, and the resultant catalyst was washed at least 3 times with 2,000 mL of deionized water, filtered and dried for over 12 hours. Thus, a catalyst for direct preparation of light olefins from synthesis gas was prepared. Thus prepared catalyst had a composition: 85 wt% Fe/5 wt% Cu/13 wt% Al, and the weight ratio of Fe-Cu-Al oxide (Fe-Cu-AlOₓ) to ferrierite zeolite was 5.

The catalyst was dried at 100 °C for over 12 hours, and calcined at 500 °C for 5 hours under air atmosphere. Finally, 3 wt% potassium was impregnated, based on the total weight of the catalyst, using K₂CO₃, to obtain a K/Fe-Cu-AlOₓ/ferrierite catalyst.

The prepared catalyst was packed in a 1/2 inch stainless steel fixed bed reactor, and reduced at 450 °C for 12 hours under hydrogen (5 vol% H₂/He) atmosphere. Reaction was carried out at 300 °C, 10 kg/cm², and a space velocity of 2,000 L/kg cat/hr, while supplying reactants at a fixed molar ratio of carbon monoxide : hydrogen : argon (internal standard) = 63.2 : 31.3 : 5.5. Reduction and reaction were carried out in the same manner as Example 2. Average value was taken after the catalytic activity was stabilized, that is, after 50 hours on stream. The result is given in Table 4.

### Example 7 (inventive)

A catalyst was prepared in the same manner as Example 6. 3 wt% zirconium was supported in a slurry phase, based on the ferrierite weight, using zirconium(IV) oxychloride octahydrate (ZrCl₂O·8H₂O). After drying in a rotary evaporator of 70 °C, calcination was carried out at 400 °C under air atmosphere to obtain a Zr-ferrierite zeolite. Then, a K/Fe-Cu-AlOx/Zr-ferrierite was prepared in the same manner as Example 5.

Reduction and reaction were carried out in the same manner as Example 2. Average value was taken after the catalytic activity was stabilized, that is, after 50 hours on stream. The result is given in Table 4.

### Example 8 (inventive)

The catalyst of Example 2 was used, and reaction was carried out at 350 °C, 10 kg/cm², and a space velocity of 2,000 L/kg cat/hr. Average value was taken after the catalytic activity was stabilized, that is, after 50 hours on stream. The result is given in Table 4.

### Comparative Example 1: Preparation of light hydrocarbons from synthesis gas

A metal mixture solution in which 21 g of iron nitrate hydrate (Fe(NO₃)₃·9H₂O), as iron precursor, 1.5 g of copper nitrate hydrate (Cu(NO₃)₂·6H₂O), as copper precursor, and 4.9 g of aluminum nitrate hydrate (Al(NO₃)₃·9H₂O), as alumina precursor, were dissolved in 400 mL deionized water was prepared. A solution in which 19.5 g of potassium carbonate was dissolved in 400 mL of deionized water was used as a precipitant. To 200 mL of deionized water in a 2,000 mL flask, the metal mixture solution and the precipitant solution prepared above were slowly added at 70 °C, and the final pH was maintained at 7.5-8.0. The mixture solution was agitated at 70 °C for about 3 hours, and the resultant catalyst was washed at least 3 times with 2,000 mL of deionized water, filtered and dried for over 12 hours. Thus, a catalyst for direct preparation of light olefins from synthesis gas was prepared. Thus prepared catalyst had a composition: 85 wt% Fe/5 wt% Cu/13 wt% Al. The catalyst was dried at 100 °C for over 12 hours, and calcined at 500 °C for 5 hours under air atmosphere. Finally, 3 wt% potassium was impregnated, based on the total weight of the catalyst, using K₂CO₃, to obtain a K/ Fe-Cu-AlOₓ catalyst. Reduction and reaction were carried out in the same manner as Example 1. Average value was taken after the catalytic activity was stabilized, that is, after 50 hours on stream. The result is given in Table 4.

**[Table 4]**

| | Total conversion of CO / Conversion of CO to CO₂ (carbon mol%) | Selectivity for C₁/C₂-C₄/C₅+ (carbon mol%) | Yield of C₂-C₄ hydrocarbons/C₂-C₄ olefins (%) |
|---|---|---|---|
| Example 2 | 68.7/29.7 | 28.6/51.6/19.8 | 20.1/9.2 |
| Example 3 (comparative) | 77.6/35.6 | 19.6/42.6/37.8 | 17.9/12.7 |
| Example 4 (comparartive) | 76.3/32.0 | 23.0/49.9/27.1 | 22.1/10.3 |
| Example 5 (comparative) | 67,4/28.6 | 16.7/40.3/43.0 | 15.6/10.7 |
| Example 6 | 91.4/38.2 | 37.3/52.3/10.4 | 27.8/9.1 |
| Example 7 | 95.5/37.8 | 33.6/52.4/14.0 | 30.2/11.5 |
| Example 8 | 81.1/34.6 | 38.7/40.1/21.2 | 18.6/11.6 |
| Comparative Example 1 | 90.7/36.7 | 4.6/19.4/76.0 | 10.5/8.9 |

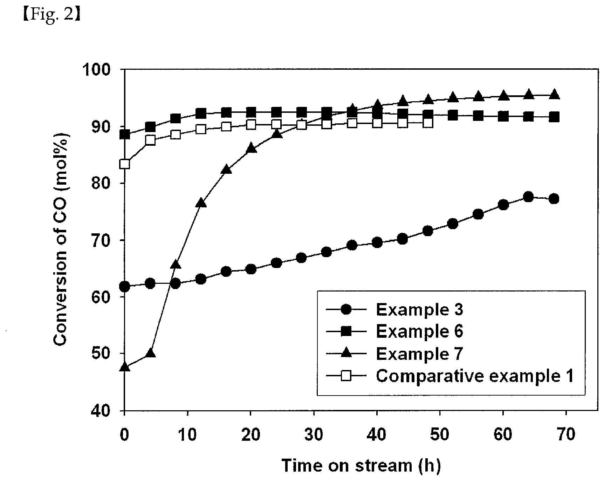

As shown in Table 4, the iron-based catalysts comprising ferrierite zeolite prepared in accordance with the present invention (Examples 2, 6-8) exhibited superior selectivity for light hydrocarbons (C₂-C₄) and superior yield of olefins and paraffins, as compared to the iron-based catalyst without comprising zeolite (Comparative Example 1). As described with reference to Fig. 1, by recycling the byproducts such as methane and carbon dioxide generated from light hydrocarbon production process to the combined reforming process, yield of light (C₂-C₄) hydrocarbons could be improved. Further, an economically feasible process could be attained because the process of thermal cracking of C5+ heavy hydrocarbons could be eliminated. As a result, the problem of broad product distribution associated with the use of the iron-based catalyst for the F-T reaction for producing liquid hydrocarbons from synthesis gas could be solved, and the yield of light hydrocarbons (C₂-C₄) could be improved. Also, as shown in Fig. 2, the zeolite-containing iron-based catalyst presented by the present invention showed very little deactivation rate with time on stream. Therefore, the catalyst has a good long-term stability and ensures a robust process for the production of light hydrocarbons from synthesis gas originated from combined reforming process.

## Claims

1. A preparation method of C₂-C₄ hydrocarbons from natural gas comprising the sequential steps of:
producing gas having a molar ratio of carbon monoxide and hydrogen being maintained at 1 :1.5-2.5, by combined reforming in which steam reforming of natural gas and carbon dioxide reforming of methane are carried out simultaneously in the presence of a Ni/alumina catalyst;
separating of CH₄/CO₂-rich, unreacted gas, and H₂/CO-rich-gas in the produced gas;
recycling the CH₄/CO₂-rich-unreacted gas to the combined reforming reaction;
producing C₂-C₄-hydrocarbons, methane and carbon dioxide as byproducts by
Fischer-Tropsch reaction at 200-700°C of carbon monoxide and hydrogen in H₂/CO-rich gas in the presence of a Fe-Cu-K/zeolite catalyst, wherein said catalyst has a specific surface area of 150-300 m²/g, comprises 10-70 wt% of Fe, 1-10 wt% of Cu and 1-10 wt% of K, based on the zeolite contents, and has been reduced under hydrogen atmosphere; and
separation of the byproducts, methane and carbon dioxide, and recycling them to the combined reforming reaction;
wherein the zeolite is ferrierite.

2. The preparation method according to claim 1, wherein the Ni/alumina catalyst is represented by the following Chemical Formula 1:
[Chemical Formula 1] NiₓCe_{y}Zr_{z}O_{x+2(y+z)}/Al₂O₃
wherein x + y + z = 1.0, x is 0.04-0.45, and y is 0.01-0.96.

3. The preparation method according to claim 1, wherein the combined reforming is carried out at 600-1,000 °C, 0.5-30 atm and a space velocity of 1,000-500,000 h⁻¹.

4. The preparation method according to claim 1, wherein
the zeolite of the Fe-Cu-K/zeolite catalyst has a Si/AI molar ratio of 2-200, and is pre-treated with a single metal precursor selected from IA, IIA, Zr, P and La or bimetal precursors by ion exchange or impregnation method.

5. The preparation method according to claim 1, wherein the Fe-Cu-K/zeolite catalyst further comprises 0-20 wt% of Al, based on the zeolite support.

6. The preparation method according to claim 1, wherein the Fischer-Tropsch reaction is carried out at 250-500 °C, 5-60 kg/cm², and a space velocity of 500-10,000 h⁻¹.

## Patentansprüche

1. Verfahren zur Herstellung von C₂-C₄-Kohlenwasserstoffen aus Erdgas, welches die aufeinanderfolgenden Schritte aufweist:
Herstellen eines Gases, welches ein molares Verhältnis von Kohlenstoffmonoxid und Wasserstoff hat, das bei 1:1,5-2,5 gehalten wird, durch kombiniertes Reformieren, wobei Dampfreformierung von Erdgas und Kohlenstoffdioxidreformierung von Methan gleichzeitig in Anwesenheit eines Ni/Aluminiumoxid-Katalysators ausgeführt werden,
Abtrennen von CH₄/CO₂-reichem, nicht umgesetztem Gas und H₂/CO-reichem Gas in dem hergestellten Gas,
Rückführen des CH₄/CO₂-reichen, nicht umgesetzten Gases zu der kombinierten Reformierungsreaktion,
Herstellen von C₂-C₄-Kohlenwasserstoffen, Methan und Kohlenstoffdioxid als Nebenprodukte durch eine Fischer-Tropsch-Reaktion bei 200-700 °C von Kohlenstoffmonoxid und Wasserstoff in H₂/CO-reichem Gas in Anwesenheit eines Fe-Cu-K/Zeolith-Katalysators, wobei der Katalysator eine spezifische Oberfläche von 150-300 m²/g hat, 10-70 Gew.-% Fe, 1-10 Gew.-% Cu und 1-10 Gew.-% K, basierend auf den Zeolithgehalten, aufweist und unter Wasserstoffatmosphäre reduziert wurde, und
Abtrennen von den Nebenprodukten, Methan und Kohlenstoffdioxid, und Rückführen derselben zu der kombinierten Reformierungsreaktion,
wobei das Zeolith Ferrierit ist.

2. Herstellungsverfahren nach Anspruch 1, wobei der Ni/Aluminiumoxid-Katalysator durch die folgende chemische Formel 1 dargestellt ist:
[chemische Formel 1] NiₓCe_{y}Zr_{z}O_{x+2(y+z)}/Al₂O₃,
wobei x+y+z=1,0, x=0,04-0,45 ist, und y=0,01-0,96 ist.

3. Herstellungsverfahren nach Anspruch 1, wobei das kombinierte Reformieren bei 600-1000 °C, 0,5-30 atm und einer Raumgeschwindigkeit von 1000-500000 h⁻¹ausgeführt wird.

4. Herstellungsverfahren nach Anspruch 1, wobei
das Zeolith des Fe-Cu-K/Zeolith-Katalysators ein molares Verhältnis Si/Al von 2-200 aufweist, und mit einem einzelnen Metallvorläufer, welcher aus IA, IIA, Zr, P und La ausgewählt ist, oder bimetallischen Vorläufern durch lonenaustausch oder ein Imprägnierungsverfahren vorbehandelt wird.

5. Herstellungsverfahren nach Anspruch 1, wobei der Fe-Cu-K/Zeolith-Katalysator weiterhin 0-20 Gew.-% Al, basierend auf dem Zeolithträger, aufweist.

6. Herstellungsverfahren nach Anspruch 1, wobei die Fischer-Tropsch-Reaktion bei 250-500 °C, 5-60 kg/cm² und einer Raumgeschwindigkeit von 500-10000 h⁻¹ ausgeführt wird.

## Revendications

1. Procédé de préparation d'hydrocarbures en C₂-C₄ à partir de gaz naturel, comprenant les étapes séquentielles suivantes :
la production d'un gaz ayant un rapport molaire du monoxyde de carbone à l'hydrogène qui est maintenu à 1:1,5-2,5, par un reformage combiné dans lequel un reformage à la vapeur de gaz naturel et un reformage au dioxyde de carbone de méthane sont réalisés simultanément en présence d'un catalyseur Ni/alumine ;
la séparation du gaz riche en CH₄/CO₂ n'ayant pas réagi et du gaz riche en H₂/CO dans le gaz produit ;
le recyclage du gaz riche en CH₄/CO₂ n'ayant pas réagi dans la réaction de reformage combiné ;
la production d'hydrocarbures en C₂-C₄, de méthane et de dioxyde de carbone en tant que sous-produits, par une réaction de Fischer-Tropsch, à 200-700°C, de monoxyde de carbone et d'hydrogène dans un gaz riche en H₂/CO en présence d'un catalyseur Fe-Cu-K/zéolite, où ledit catalyseur possède une surface spécifique de 150-300 m²/g, comprend 10-70% en poids de Fe, 1-10% en poids de Cu et 1-10% en poids de K, basés sur les teneurs en zéolite, et a été réduit sous atmosphère d'hydrogène ; et
la séparation des sous-produits, méthane et dioxyde de carbone, et leur recyclage dans la réaction de reformage combiné ;
dans lequel la zéolite est une ferriérite.

2. Procédé de préparation selon la revendication 1, dans lequel le catalyseur Ni/alumine est représenté par la formule chimique 1 suivante :
[Formule chimique 1] NiₓCe_{y}Zr_{z}O_{x+2(y+z)}/Al₂O₃
dans laquelle x + y + z =1,0, x est égal à 0,04-0,45, et y est égal à 0,01-0,96.

3. Procédé de préparation selon la revendication 1, dans lequel le reformage combiné est réalisé à 600-1000°C, 0,5-30 atm et at une vitesse spatiale de 1000-500 000 h⁻¹.

4. Procédé de préparation selon la revendication 1, dans lequel la zéolite du catalyseur Fe-Cu-K/zéolite a un rapport molaire Si/Al de 2-200, et est prétraitée à l'aide d'un précurseur monométallique choisi parmi IA, IIA, Zr, P et La ou des précurseurs bimétalliques par un procédé d'échange d'ions ou d'imprégnation.

5. Procédé de préparation selon la revendication 1, dans lequel le catalyseur Fe-Cu-K/zéolite comprend en outre 0-20% en poids d'Al, basé sur le support de zéolite.

6. Procédé de préparation selon la revendication 1, dans lequel la réaction de Fischer-Tropsch est réalisée à 250-500°C, 5-60 kg/cm², et une vitesse spatiale de 500-10 000 h⁻¹.
